# EUROPÄISCHE PATENTSCHRIFT

(11) **EP 0 890 565 B1**
(45) Veröffentlichungstag und Bekanntmachung des Hinweises auf die Patenterteilung: **03.04.2002**
(21) Anmeldenummer: 98111053.9
(22) Anmeldetag: 17.06.1998
(51) Int. Cl.: C07C 45/00, C07C 49/417

(54) **Verfahren zur Herstellung von Cyclohexanonen durch Hydrierung der entsprechenden Phenole in Gegenwart von Wasser**
Process for preparing cyclohexanones by hydrogenation of the corresponding phenols in presence of water
Procédé pour la préparation de cyclohéxanones par hydrogénation des phénols correspondants en présence d'eau

(30) Priorität: 30.06.1997 DE 19727712
(43) Veröffentlichungstag der Anmeldung: 13.01.1999
(73) Patentinhaber: BAYER AG, 51368 Leverkusen (DE)
(72) Erfinder: Kiel, Wolfgang, Dr., 51519 Odenthal (DE)

(56) Entgegenhaltungen:
- DE-A- 2 752 291
- DE-A- 2 909 780
- US-A- 3 998 884
- US-A- 4 200 553
- DATABASE WPI Section Ch, Week 8207 Derwent Publications Ltd., London, GB; Class B05, AN 82-12956E XP002077120 & JP 57 004 932 A (UBE IND LTD)

## Beschreibung

Die vorliegende Erfindung betrifft ein vorteilhaftes Verfahren zur Hydrierung von Phenolen zu den entsprechenden Cyclohexanonen unter Verwendung von Palladium-auf-Kohle-Katalysatoren.

Verfahren zur Hydrierung von Phenolen zu Cyclohexanonen an Palladium-auf-Kohle-Katalysatoren sind grundsätzlich bekannt (siehe z.B. DE-A 2 909 780, DE-A 2 530 759 und US-A 2 829 166).

In der Regel wird zum Erreichen einer guten Selektivität ein Wasserstoffdruck von weniger als 20 bar und eine Reaktionstemperatur im Bereich von 120 bis 250°C angestrebt und dem Hydriergemisch eine alkalisch reagierende Verbindung zugefügt. Der Katalysator kann gegebenenfalls mit Alkalimetallionen wie Na- oder K-Ionen behandelt werden (siehe z.B. DE-AS 1 144 267). Der Einsatz von Phenolaten und Bicarbonaten ist in der FR-A 2 372 136 beschrieben.

Nach der DE-A 2 909 780 wird zur Hydrierung von p-tert.-Amylphenol Natriumcarbonat, Borax und/oder Natriumacetat eingesetzt. Gleichzeitig kommt hier zum Ausdruck, daß der Einsatz von Palladium-Katalysatoren mit Metalloberflächen von 15 m²/g und mehr erfolgversprechend ist. Bevorzugt werden Palladium-auf-Kohle-Katalysatoren eingesetzt, die 5 Gew.-% Palladium enthalten, bezogen auf einen Träger, der eine Oberfläche von 800 m²/g aufweist.

Bei der Anwendung dieser bekannten Verfahren, insbesondere auch bei deren Ausdehnung auf weitere substituierte Phenole, stellt man fest, daß die Hydrierergebnise (z.B. Ausbeute an gewünschten Cyclohexanonen) und die notwendigen Hydrierzeiten bei wiederholten Ansätzen unter gleichen Bedingungen stark schwanken (s. Beispiele 14 bis 19).

Da Cyclohexanone in vielen Fällen als Ausgangsverbindungen für Pharmazeutika und Pflanzenschutzmittel Verwendung finden, muß eine hohe Reinheit gewährleistet sein. Speziell im Falle schwerabtrennbarer Nebenkomponenten, liegt daher der Wert eines Herstellungsverfahrens in dem Erreichen einer möglichst hohen Selektivität mit gleichzeitiger guter Reproduzierbarkeit der Ergebnisse. Außerdem treten bei stark variierenden Hydrierergebnissen und -zeiten Probleme in der Reaktionsführung und bei der Aufarbeitung der Reaktionsgemische auf.

Es wurde nun ein Verfahren zur Herstellung von Cyclohexanonen der Formel (I) gefunden, 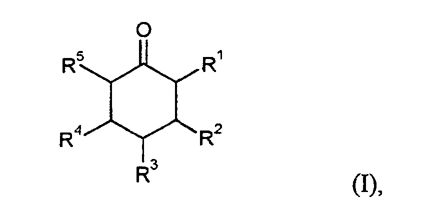 in der
- R¹, R², R³, R⁴ und R⁵: unabhängig voneinander jeweils für Wasserstoff, Hydroxy, C₁-C₁₀-Alkyl, C₃-C₆-Cycloalkyl, C₆-C₁₀-Aryl, C₆-C₁₀-Aryl-CH₂-, C₆-C₁₀-Aryl-O-, C₁-C₁₀-Alkoxy, C₃-C₈-Cycloalkoxy, C₁-C₄-Alkylamino, Di-C₁-C₄-alkylamino, C₁-C₄-Acylamino, COOR⁶ mit R⁶ = Wasserstoff oder C₁-C₄-Alkyl oder R⁷-CH₂- mit R⁷ = Hydroxy, C₁-C₄-Alkoxy, C₁-C₄-Alkylamino oder Di-C₁-C₄-alkylamino bedeuten,
durch Hydrierung von Phenolen der Formel (II) 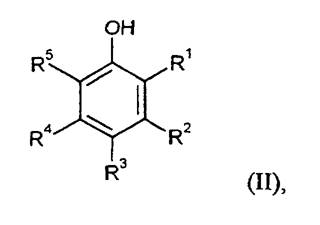 in der R¹ bis R⁵ die bei Formel (I) angegebene Bedeutung haben,
in Gegenwart eines Palladium-auf-Kohle-Katalysators bei 100 bis 250°C und 1 bis 20 bar Wasserstoffdruck, das dadurch gekennzeichnet ist, daß man den Katalysator mit einer Basenkomponente und 20 bis 200 Gew.-% Wasser (bezogen auf die Basenkomponente) vermischt und dieses Gemisch in die Hydrierung einsetzt.

In den Formeln (I) und (II) stehen R¹, R², R³, R⁴ und R⁵ unabhängig voneinander vorzugsweise für Wasserstoff, Hydroxy, C₁-C₆-Alkyl, C₁-C₆-Alkoxy oder C₁-C₄-Acylamino. Außerdem ist es bevorzugt, daß 1 bis 4 der Reste R¹ bis R⁵ von Wasserstoff verschieden sind.

Beispiele für C₁-C₆-Alkyl sind Methyl, Ethyl, i-Propyl, t-Butyl und Pentyl. Beispiele für C₁-C₆-Alkoxy sind Methoxy, Ethoxy und i-Propoxy. Ein Beispiel für C₁-C₄-Acylamino ist Acetylamino.

Besonders bevorzugt stehen R¹, R², R⁴ und R⁵ für Wasserstoff und R³ für Hydroxy, C₁-C₆-Alkyl, C₁-C₆-Alkoxy oder C₁-C₄-Acylamino.

Die Palladium-auf-Kohle-Katalysatoren können z.B. 1 bis 15 Gew.-% Palladium auf einer beliebigen Kohle enthalten. Vorzugsweise enthalten solche Katalysatoren 2 bis 12 Gew.-% Palladium. Derartige Katalysatoren sind im Handel erhältlich. Häufig werden sie in wasserfeuchter Form angeboten und können auch in dieser wasserfeuchten Form eingesetzt werden. Bezogen auf das Phenol der Formel (II) kann man beispielsweise 0,001 bis 1 Gew.-% Katalysator (berechnet als Pd-Metall) einsetzen. Bevorzugt beträgt diese Menge 0,05 bis 0,2 Gew.-%.

Die Reaktionstemperatur liegt vorzugsweise bei 150 bis 200°C und der Wasserstoffdruck vorzugsweise bei 3 bis 8 bar.

Es ist ein wesentliches Merkmal der vorliegenden Erfindung, daß man den Katalysator nicht als solchen oder in wasserfeuchter Form einsetzt, sondern ihn vorher mit einer Basenkomponente und 20 bis 200 Gew.-% Wasser (bezogen auf die Basenkomponente) vermischt. Als Basenkomponente kommen insbesondere in wäßrigem Milieu basisch reagierende Alkalisalze infrage. Bevorzugt sind Borax (Na₂B₄O₇x10H₂O), Natriumacetat und Dinatriumhydrogenphosphat. Bezogen auf den Palladium-auf-Kohle-Katalysator kann man beispielsweise 5 bis 50 Gew.-% Basenkomponente einsetzen. Vorzugsweise beträgt diese Menge 10 bis 20 Gew.-%.

Die Wassermenge bezogen auf die Basenkomponente beträgt vorzugsweise 50 bis 150 Gew.-%. Bei der einzusetzenden Wassermenge wird in der Basenkomponente eventuell vorhandenes Kristallwasser nicht berücksichtigt. Ebenso wird das Wasser nicht berücksichtigt, das evtl. für die Befeuchtung des Katalysators zu seiner sichereren Handhabung verwendet wurde. Es ist vorteilhaft, zunächst die Basenkomponente mit Wasser zu vermischen, wobei es unerheblich ist, ob sich die Basenkomponente vollständig löst oder nicht, und dann das Basenkomponente/Wasser-Gemisch mit dem trockenen oder wasserfeuchten Palladium-auf-Kohle-Katalysator zu vermischen.

Man kann das erfindungsgemäße Verfahren in Anwesenheit oder Abwesenheit von Lösungsmitteln durchführen. Wenn das eingesetzte Phenol der Formel (II) einen Schmelzpunkt unterhalb der Hydriertemperatur hat, ist es bevorzugt, mit geschmolzenem Phenol der Formel (II) und ohne Zusatz von Lösungsmitteln zu arbeiten. Beim Einsatz von Phenolen der Formel (II) mit höheren Schmelzpunkten muß man ein Lösungsmittel zusetzen, beispielsweise einen Alkohol oder Ether.

Das erfindungsgemäße Verfahren kann beispielsweise in Rühr- oder Schlaufenreaktoren durchgeführt werden.

Das erfindungsgemäße Verfahren hat die Vorteile, daß die Hydrierzeiten bei gleichen Ansätzen nahezu gleich sind und mit ihm die gewünschten Cyclohexanone der Formel (I) bei gleichen Bedingungen selektiv in stets praktisch gleich guten Ausbeuten erhalten werden. Beides führt zu hohen Kosteneinsparungen bei der Aufarbeitung der Reaktionsgemische.

### Beispiele

### Beispiele 1 bis 5

0,5 g Borax und 0,5 g Wasser wurden vermischt und dann 6 g eines wasserfeuchten Katalysators (Wassergehalt 50 Gew.-%) zugemischt, der 5 Gew.-% Palladium auf Kohle enthielt. Das so erhaltene Gemisch wurde zu 300 g geschmolzenem p-tert.-Butylphenol gegeben und dieses Reaktionsgemisch in einem Rührreaktor bei 150 bis 180°C und 3 bis 8 bar Wasserstoffdruck hydriert bis kein Wasserstoff mehr aufgenommen wurde. Es wurden nacheinander 5 Ansätze (=Beispiele 1 bis 5) durchgeführt. Die Ergebnisse sind aus Tabelle 1 ersichtlich.

**Tabelle 1**

| Beispiel Nr. | Hydrierzeit (min) | Gehalt des Reaktionsgemisches an p-tert.-Butylcyclohexanon (Gew.-%) |
|---|---|---|
| 1 | 83 | 93,2 |
| 2 | 89 | 92,2 |
| 3 | 85 | 91,4 |
| 4 | 88 | 91,8 |
| 5 | 83 | 91,7 |

### Beispiele 6 und 7

Es wurde gearbeitet wie bei Beispiel 1, jedoch wurden statt Borax 0,5 g Dinatriumhydrogenphosphat (Beispiel 6) bzw. 0,5 g Natriumacetat (Beispiel 7) eingesetzt. Die Ergebnisse sind aus Tabelle 2 ersichtlich, in der auch nochmal das Beispiel 1 enthalten ist.

**Tabelle 2**

| Beispiel Nr. | Basenkomponente | Hydrierzeit (min) | Gehalt des Reaktionsgemisches an p-tert.-Butylcyclohexanon (Gew.-%) |
|---|---|---|---|
| 1 | Borax | 83 | 93,2 |
| 6 | Na₂HPO₄ | 48 | 88,0 |
| 7 | CH₃COONa | 41 | 90,1 |

### Beispiele 8 bis 13

Es wurde gearbeitet wie bei Beispiel 1, jedoch wurden 6 g eines wasserfeuchten Katalysators verwendet (Wassergehalt 50 Gew.-%), der 10 Gew.-% Palladium auf Kohle enthielt (Beispiel 8). Der Katalysator wurde danach abgetrennt und erneut in einem Ansatz unter gleichen Bedingungen wiederverwendet. Diese Wiederverwendung wurde insgesamt 5 mal nacheinander durchgeführt (Beispiele 9 bis 13). Die Ergebnisse sind aus Tabelle 3 ersichtlich, in der auch nochmal das Beispiel 1 enthalten ist.

**Tabelle 3**

| Beispiel Nr. | Katalysator (Gew.-% Pd auf Kohle | Hydrierzeit (min) | Gehalt des Reaktionsgemisches an p-tert.-Butylcyclohexanon (Gew.-%) |
|---|---|---|---|
| 1 | 5 | 83 | 93,2 |
| 8 | 10 | 52 | 90,6 |
| 9 | 10 | 46 | 91,3 |
| 10 | 10 | 44 | 92,3 |
| 11 | 10 | 44 | 91,2 |
| 12 | 10 | 42 | 92,1 |
| 13 | 10 | 40 | 90,1 |

### Beispiele 14 bis 19 (zum Vergleich)

Es wurde gearbeitet wie in Beispiel 1, jedoch wurde Borax ohne Wasser und der Katalysator getrennt in geschmolzenes p-tert.-Butylphenol gegeben. Es wurden nacheinander 6 Ansätze unter gleichen Bedingungen durchgeführt. Die Ergebnisse sind aus Tabelle 4 ersichtlich.

**Tabelle 4**

| Beispiel Nr. | Hydrierzeit (min) | Gehalt des Reaktionsgemisches an p-tert.-Butylcyclohexanon (Gew.-%) |
|---|---|---|
| 14 | 51 | 91,4 |
| 15 | 32 | 86,5 |
| 16 | 224 | 83,7 |
| 17 | 72 | 90,6 |
| 18 | 92 | 29,2 |
| 19 | 1126 | 64,6 |

## Patentansprüche

1. Verfahren zur Herstellung von Cyclohexanonen der Formel 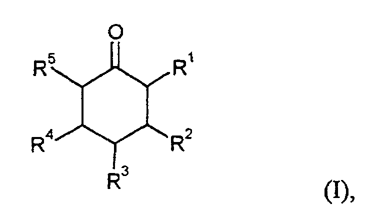 in der
R¹, R², R³, R⁴ und R⁵ unabhängig voneinander jeweils für Wasserstoff, Hydroxy, C₁-C₁₀-Alkyl, C₃-C₆-Cycloalkyl, C₆-C₁₀-Aryl, C₆-C₁₀-Aryl-CH₂-, C₆-C₁₀-Aryl-O-, C₁-C₁₀-Alkoxy, C₃-C₈-Cycloalkoxy, C₁-C₄-Alkylamino, Di-C₁-C₄-alkylamino, C₁-C₄-Acylamino, COOR⁶ mit R⁶ = Wasserstoff oder C₁-C₄-Alkyl oder R⁷-CH₂- mit R⁷ = Hydroxy, C₁-C₄-Alkoxy, C₁-C₄-Alkylamino oder Di-C₁-C₄-alkylamino stehen,
durch Hydrierung von Phenolen der Formel (II) 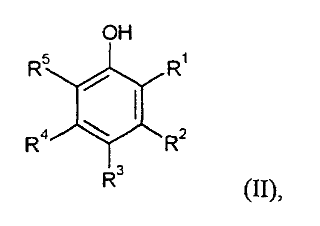 in der R¹ bis R⁵ die bei Formel (I) angegebene Bedeutung haben,
in Gegenwart eines Palladium-auf-Kohle-Katalysators bei 100 bis 250°C und 1 bis 20 bar Wasserstoffdruck, **dadurch gekennzeichnet, daß** man den Katalysator mit einer Basenkomponente und 20 bis 200 Gew.-% Wasser (bezogen auf die Basenkomponente) vermischt und dieses Gemisch in die Hydrierung einsetzt.

2. Verfahren nach Anspruch 1, **dadurch gekennzeichnet, daß** in den Formeln (I) und (II) R¹, R², R³, R⁴ und R⁵ unabhängig voneinander für Wasserstoff, Hydroxy, C₁-C₆-Alkyl, C₁-C₆-Alkoxy oder C₁-C₄-Acylamino stehen und 1 bis 4 der Reste R¹ bis R⁵ von Wasserstoff verschieden sind.

3. Verfahren nach Ansprüchen 1 und 2, **dadurch gekennzeichnet, daß** in den Formeln (I) und (II) R¹, R², R⁴ und R⁵ für Wasserstoff und R³ für Hydroxy, C₁-C₆-Alkyl, C₁-C₆-Alkoxy oder C₁-C₄-Acylamino stehen.

4. Verfahren nach Ansprüchen 1 bis 3, **dadurch gekennzeichnet, daß** der Palladium-auf-Kohle-Katalysator 1 bis 15 Gew.-% Palladium enthält.

5. Verfahren nach Ansprüchen 1 bis 4, **dadurch gekennzeichnet**, das bezogen auf das Phenol der Formel (II) 0,001 bis 1 Gew.-% Katalysator (gerechnet als Pd-Metall) eingesetzt werden.

6. Verfahren nach Ansprüchen 1 bis 5, **dadurch gekennzeichnet, daß** als Basenkomponente Borax, Natriumacetat oder Dinatriumhydrogenphosphat eingesetzt wird.

7. Verfahren nach Ansprüchen 1 bis 6, **dadurch gekennzeichnet, daß** die Basen-komponente in einer Menge von 5 bis 50 Gew.-%, bezogen auf den Palladium-auf-Kohle-Katalysator, eingesetzt wird.

8. Verfahren nach Ansprüchen 1 bis 7, **dadurch gekennzeichnet, daß** die Wassermenge, bezogen auf die Basenkomponente, 50 bis 150 Gew.-% beträgt.

9. Verfahren nach Ansprüchen 1 bis 8, **dadurch gekennzeichnet, daß** man zunächst die Basenkomponente mit Wasser vermischt und dann das Basenkomponente/Wasser-Gemisch mit Katalysator vermischt.

## Claims

1. Process for the preparation of cyclohexanones of the formula 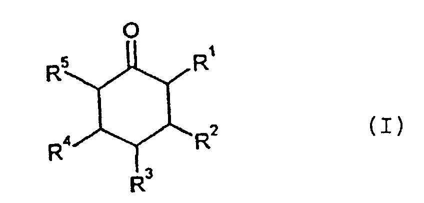 in which
R¹, R², R³, R⁴ and R⁵ each independently of the others represents hydrogen, hydroxy, C₁-C₁₀-alkyl, C₃-C₆-cycloalkyl, C₆-C₁₀-aryl, C₆-C₁₀-aryl-CH₂-, C₆-C₁₀-aryl-O-, C₁-C₁₀-alkoxy, C₃-C₈-cycloalkoxy, C₁-C₄-alkylamino, di-C₁-C₄-alkylamino, C₁-C₄-acylamino, COOR⁶ wherein R⁶ = hydrogen or C₁-C₄-alkyl, or R⁷-CH₂- wherein R⁷ = hydroxy, C₁-C₄-alkoxy, C₁-C₄-alkylamino or di-C₁-C₄-alkylamino,
by hydrogenation of phenols of formula (II) 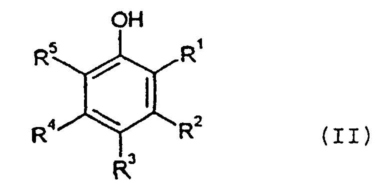 in which R¹ to R⁵ are as defined for formula (I),
in the presence of a palladium-on-carbon catalyst at from 100 to 250°C and a hydrogen pressure of from 1 to 20 bar, **characterised in that** the catalyst is mixed with a base component and from 20 to 200 wt.% water (based on the base component) and that mixture is used in the hydrogenation.

2. Process according to claim 1, **characterised in that** in formulae (I) and (II) R¹, R², R³, R⁴ and R⁵ each independently of the others represents hydrogen, hydroxy, C₁-C₆-alkyl, C₁-C₆-alkoxy or C₁-C₄-acylamino, and from 1 to 4 of the radicals R¹ to R⁵ are other than hydrogen.

3. Process according to claims 1 and 2, **characterised in that** in formulae (I) and (II) R¹, R², R⁴ and R⁵ represent hydrogen and R³ represents hydroxy, C₁-C₆-alkyl, C₁-C₆-alkoxy or C₁-C₄-acylamino.

4. Process according to claims 1 to 3, **characterised in that** the palladium-on-carbon catalyst contains from 1 to 15 wt.% palladium.

5. Process according to claims 1 to 4, **characterised in that** from 0.001 to 1 wt.% catalyst (calculated as Pd metal) is used, based on the phenol of formula (II).

6. Process according to claims 1 to 5, **characterised in that** borax, sodium acetate or disodium hydrogen phosphate is used as the base component.

7. Process according to claims 1 to 6, **characterised in that** the base component is used in an amount of from 5 to 50 wt.%, based on the palladium-on-carbon catalyst.

8. Process according to claims 1 to 7, **characterised in that** the amount of water, based on the base component, is from 50 to 150 wt.%.

9. Process according to claims 1 to 8, **characterised in that** the base component is first mixed with water and then the base component/water mixture is mixed with catalyst.

## Revendications

1. Procédé pour la préparation de cyclohexanones de formule 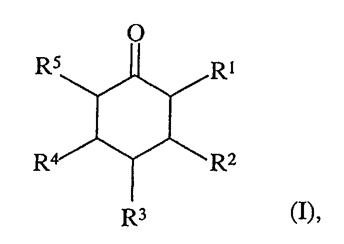 dans laquelle
R¹, R², R³, R⁴ et R⁵ représentent chacun, indépendamment les uns des autres, l'hydrogène, un groupe hydroxy, alkyle en C₁-C₁₀, cycloalkyle en C₃-C₆, aryle en C₆-C₁₀, (aryle en C₆-C₁₀)-CH₂-, (aryle en C₆-C₁₀)-O-, alcoxy en C₁-C₁₀, cycloalcoxy en C₃-C₈, alkylamino en C₁-C₄, di(alkyle en C₁-C₄)amino, acylamino en C₁-C₄, COOR⁶ dans lequel R⁶ = hydrogéne ou alkyle en C₁-C₄, ou R⁷-CH₂- dans lequel R⁷ = hydroxy, alcoxy en C₁-C₄, alkylamino en C₁-C₄ ou di-(alkyle en C₁-C₄)amino,
par hydrogénation de phénols de formule (II) 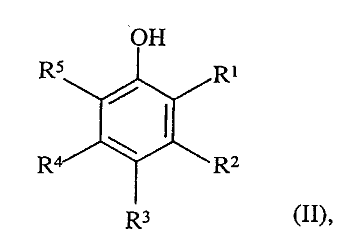 dans laquelle R¹ à R⁵ ont les significations indiquées en référence à la formule (I), en présence d'un catalyseur au palladium sur charbon à des températures de 100 à 250°C sous une pression d'hydrogène de 1 à 20 bars, **caractérisé en ce que** l'on mélange le catalyseur avec un composant basique et 20 à 200 % en poids d'eau (par rapport au composant basique) et on utilise ce mélange à l'hydrogénation.

2. Procédé selon la revendication 1, **caractérisé en ce que**, dans les formules (I) et (II), R¹, R², R³, R⁴ et R⁵ représentent chacun, indépendamment les uns des autres, l'hydrogène, un groupe hydroxy, alkyle en C₁-C₆, alcoxy en C₁-C₆ ou acylamino en C₁-C₄ et un à quatre des symboles R¹ à R⁵ ont une signification autre que l'hydrogène.

3. Procédé selon les revendications 1 et 2, **caractérisé en ce que**, dans les formules (I) et (II), R¹, R², R⁴ et R⁵ représentent l'hydrogène et R³ un groupe hydroxy, alkyle en C₁-C₆, alcoxy en C₁-C₆ ou acylamino en C₁-C₄.

4. Procédé selon les revendications 1 à 3, **caractérisé en ce que** le catalyseur au palladium sur charbon contient de 1 à 15 % en poids de palladium.

5. Procédé selon les revendications 1 à 4, **caractérisé en ce que**, par rapport au phénol de formule (II), on utilise de 0,001 à 1 % en poids de catalyseur (exprimé en palladium métallique).

6. Procédé selon les revendications 1 à 5, **caractérisé en ce que** l'on utilise en tant que composant basique le borax, l'acétate de sodium ou l'hydrogénophosphate disodique.

7. Procédé selon les revendications 1 à 6, **caractérisé en ce que** le composant basique est mis en oeuvre en quantité de 5 à 50 % du poids du catalyseur au palladium sur charbon.

8. Procédé selon les revendications 1 à 7, **caractérisé en ce que** la quantité d'eau représente de 50 à 150 % du poids du composant basique.

9. Procédé selon les revendications 1 à 8, **caractérisé en ce que** l'on mélange d'abord le composant basique avec l'eau puis on ajoute le catalyseur au mélange composant basique/eau et on mélange.
